# EUROPEAN PATENT APPLICATION

(11) **EP 1 759 695 A1**
(43) Date of publication of application: **07.03.2007**
(21) Application number: 06121128.0
(22) Date of filing: 22.03.2004
(51) Int. Cl.: A61K 31/19, A23L 1/29, A23L 1/304, A61P 7/08, A61P 9/00

(54) **Lactate containing pharmaceutical composition and uses thereof**

(30) Priority: 01.05.2003 ID 20030213
(62) Divisional of application: 04722501.6
(71) Applicant: Innogene Kalbiotech Pte. Ltd., Singapore 048423 (SG)
(72) Inventor: Leverve, Xavier M., 38660, La Terrasse (FR); Iqbal, Mustafa, Jakarta (IN)
(74) Representative: Viering, Jentschura & Partner

(57) **Abstract**

The invention relates to a pharmaceutical composition containing 250 to 2400 millimoles per liter of lactate or lactic acid, 2 to 10 millimoles per liter of potassium cation and optionally 2 to 5 millimoles per liter of calcium cation. The invention also relates to pharmaceutical uses of this composition. The invention also relates to pharmaceutical uses of a composition having a lactic acid or lactate concentration between 250 to 2400 millimoles per liter.

## Description

The present invention relates to a lactate containing pharmaceutical composition, to a method of preparing the pharmaceutical composition as well as to various medical and therapeutic uses of this composition. In particular, the invention relates to a pharmaceutical composition and the use thereof in the treatment of diseases and disorders such as cardiovascular diseases, brain disorders, organ failure, obesity, acute hemodynamic distress due to medical and surgery, septic shock or obesity. In one particular aspect, the invention also relates to the use of a hypertonic lactate solution for the treatment of brain disorders.

Lactic acid as such or in form of its anion; the lactate anion or salts thereof, has found rather widespread application in the pharmaceutical field. Traditionally, the lactate anion is used as buffering agent in compositions for dialysis, see for example, Chung et al. Perit. Dial. Int. 2000, 20 Suppl. 5: S57-67, or US patent 6,610,206. Lactate is also an ingredient in Ringer's lactate, an aqueous solution that is isotonic with the human blood (containing 130 mmol/l Na⁺, 5.4 mmol/l K⁺, 1.85 mmol/l Ca²⁺, 27 mmol/l lactate and 112 mmol/Cl⁻), used as physiological saline solution for intravenous infusion in hypovolemia. In addition, lactate has been described in US patent 5,100,677 as one permanent mono-anionic metabolite selected from the group of pyruvate, lactate, d-betahydroxybuytrate, acetoacetate, that can be employed for fluid therapy. According to this patent a solution containing 0.01 to 2400 mmol/l L-lactate is suitable for parental, oral, dialysis and irrigation therapy. Specific examples of conditions that can be treated according to this US patent are acidosis, dehydration, blood electrolyte depletion, shock, malnutrition and uremia.

More recently, the lactate anion has also been the subject of research in patients undergoing cardiac surgery. In this study, the metabolic and hemodynamic effects of a 1 M hypertonic lactate solution (consisting of 90 g lactate and 23 g sodium per liter) was investigated in postoperative patients who underwent elective coronary artery bypass grafting (CABG) (Mustafa, I. and Leverve, X.M. Shock, 18, 306-310, 2002). The authors concluded from this study that hypertonic lactate solution is safe and well tolerated in patients that undergo this kind of surgery.

However, despite these promising results it would be still desirable to have a lactate containing composition that is easy to manufacture, easy to use and suitable for a great variety of therapeutic applications. Therefore, it is an object of the invention to provide such a composition.

This object is solved among others by the pharmaceutical composition having the feature of the respective independent claim. Such a composition is a pharmaceutical composition containing 250 to 2400 millimoles per liter of lactic acid or lactate; and 2 to 10 millimoles per liter of potassium. In addition, the invention is also directed to the use of a hypertonic lactate composition, i.e. a composition having a lactic acid or lactate concentration of 250 to 2400 millimoles per liter, for the treatment of brain disorder such as traumatic brain injury, cerebral ischemia or non-traumatic brain injury. Accordingly, a suitable pharmaceutical composition that is used for the treatment of such brain disorders may only contain lactate or lactic acid in the specified concentration range as the only (pharmaceutically active) component, i.e. it may not contain potassium.

The invention is based on the surprising finding that hypertonic lactate containing compositions (compositions that comprise lactate as active ingredient in concentrations such as described here) have highly versatile applications and a high effectiveness in such diverse therapeutic indications such as the treatment of an elevated intracranial blood pressure (ICP) or brain edema which can be caused by traumatic brain injury or the treatment of acute hemodynamic distress caused by polytrauma, shock or post-operative situations, for example.

In preferred embodiments of the invention, the concentration of lactic acid or the lactate anion is in the range of about 350 to about 2000 mmol, or about 400 to about 1500 mmol or in the range of 500 to about 1500 millimoles per liter. In other preferred embodiments the concentration of lactic acid or the lactate anion is in the range of about 800 to about 1200 millimoles per liter. In some embodiments a concentration of the lactic acid or lactate of about 500 or about 1000 millimoles per liter has found to be particularly suitable. However, depending on the concrete application and also on the severity of the condition and the individual to be treated, any suitable lactate concentration within the range of 250 to 2400 mmol/L, can be chosen. Accordingly, any lactate concentration within this range, for example, 350, 500, 800, or 2200 mmol lactate can be used in combination with any concentration of the other ingredients which may be present in the composition of the invention, for example, any potassium concentration which is in the range of 2 to 10 millimoles or a calcium concentration that is within the range of about 2 to about 5 millimoles per liter (see below).

It should also be noted in this respect, that the term "lactate" comprises both enantiomeric forms, i.e. D-lactate as well as the L-lactate, wherein L-Lactate is preferred. However, as long as the D-lactate is present in amounts which do not have an adverse or even toxic effect on the patient to be treated, a mixture of L- and D-lactate can also be used in the invention. The term "lactic acid" accordingly also includes D-lactic and L-lactic acid and further includes polymeric or oligomeric forms of lactic acid such a polylactic acid (polylactate). Furthermore, derivates of lactic acid such as esters of lactic acid are also within the meaning of the term "lactic acid". Examples of such esters are methyl lactate, ethyl lactate, or esters of lactate acid with polyols such as glycerol to name a few. Furthermore, the use of mixtures of lactic acid, lactic acid derivates such as esters thereof, and lactate is also within the scope of the invention, i.e. a pharmaceutical composition may contain lactic acid, polylactic acid and a lactate salt.

In order to achieve electroneutrality of the composition of the invention (especially, once the composition is in present as fluid), a cation such as ammonium, dimethylammonium, diethylammonium, sodium or a mixture of such cations is also present in the composition, if lactate is used. Preferably, sodium is used in some embodiments as counter-ion for the lactate anion, i.e. in those cases the concentration of sodium is identical to the chosen lactate concentration. For this reason, sodium lactate is a preferred compound used in preparing a composition of the invention. If lactic acid is used, no other cation (except protons or H₃O⁺, which result from the dissociation of lactic acid) needs to be present in order to achieve electroneutrality. However, if wanted, physiologically useful cations as described below can be present in addition to the lactic acid, if lactic acid is used as active ingredient in the present invention.

In addition, the composition also contains potassium. The presence of potassium has been found to be in particular useful in order to prevent a hypokalemia which may be caused by the treatment with hypertonic sodium lactate alone. In some embodiments of the composition of the invention, the potassium concentration is in the range ot 2.5 to 6 millimoles per litre, with a potassium concentration of about 3.5 mmol or about 4 mmol/L being presently preferred in some embodiments.

The composition of the invention may also contain calcium in a concentration of about 2 to about 5 millimoles per liter of calcium. In some embodiments of the composition of the invention, in which calcium is used, a calcium concentration in the range of 2.5 to 4 millimoles per liter is presently preferred. A concentration of 2.7 millimoles/litre is particularly suitable for some applications. The combination of the metabolisable lactate anion and the calcium ion in the specified concentration range has been found to powerfully increase the hemodynamic function of a patient. For example, the combined presence of lactate and calcium significantly increase the cardiac contraction (due to an inotropic effect). In addition, this combination allows to relax tone both in the general circulation of in the pulmonary vascularisation (decrease in vascular resistance), which results in a significant increase in cardiac output, even in patients with cardiac failure, for example (cf. Example 3). In this respect, it should be noted that the composition described here has a remarkable anti-ischemic/antioxidant effect, and can thus be used for improving the recovery of affected patients after an ischemia-reperfusion injury (cf. Example 4). In this connection, it should further be noted that the composition of the invention also possesses a significant volume effect (fluid replenishment) rendering it an attractive agent for patients requiring fluid infusion for resuscitation, for example. Surprisingly, this volume effect is stronger than the hemodynamic effect of known crystalloid solutions such as a mannitol solution. It has been shown that only 50 to 70 % of the volume of such a known solution is necessary to obtain the same hemodynamic effect (cf. Example 2) Such a reduction yields in a significant decrease of the stress the treatment represent for the patient, for example, as less fluid has to be purified by the kidney or as the risks of producing swellings and edema decreases. Accordingly, the composition of the invention is able to significantly decrease possible side effects of the treatment. Finally, the composition of the invention has surprisingly been found to be able to strongly decrease the intracranial pressure during an acute increase (due to a brain trauma). This increase has been found to be more pronounced and more prolonged that the effect of mannitol, the standard treatment for this disorder so far (cf. Example 2).

In addition to the above-described components, the composition according to the invention typically comprises chloride (CI) as negatively charged counter-ion for the potassium and the calcium cations.

In accordance with the above disclosure, the composition of the invention is preferably used as an aqueous solution.

In one presently particularly preferred embodiment, the composition of the invention contains the above-mentioned ingredients in the following concentrations:
1000 millimoles per liter lactate,
9.4 millimoles per liter chloride (Cl),
4 millimoles per liter potassium (K),
2.7 millimoles per liter calcium (Ca), and
1000 millimoles per liter sodium (Na).

In another presently particularly preferred embodiment the composition contains these ingredients in the following concentrations:
1000 millimoles per liter lactate,
8.9 millimoles per liter chloride (Cl),
3.5 millimoles per liter potassium (K)
2.7 millimoles per liter calcium (Ca), and
1000 millimoles per liter sodium (Na).

In other presently particularly preferred embodiments, the following concentrations are used in the composition:
500 millimoles per liter lactate,
8.9 to 9.4 millimoles per liter chloride (Cl),
3.5 to 4 millimoles per liter potassium (K),
2.7 millimoles per liter calcium (Ca), and
500 millimoles per liter sodium (Na).

Yet another example of a presently preferred embodiment is a composition having the following concentrations:
750 millimoles per liter lactate,
8.9 to 9.4 millimoles per liter chloride (Cl),
3.5 to 4 millimoles per liter potassium (K),
2.7 millimoles per liter calcium (Ca), and
750 millimoles per liter sodium (Na).

The composition may further contain other ingredients, for example, further physiologically relevant cations such as magnesium or zinc. Magnesium may be present in a concentration of up to about 3 or 4 mmol/litre. The composition may also contain phosphate, in addition to such physiologically relevant cations or independent from their presence. The phosphate may be added in any suitable form, for example, as monohydrogen or dihydrogen phosphate. Examples of suitable phosphate salts are NaH₂PO₄ and Na₂HPO₄. If present, the phosphate is typically employed in a concentration up to 5 mmol/liter. A further compound that may also be added to the composition of the invention in a concentration of up to about 5 mmol/liter is ATP. ATP may be used in the form of its magnesium salt.

Other suitable additives that may be included in the composition are agents that exert an osmotic effect (osmolytes and oncotic agents) and thus can further increase the osmotic effect of the composition of the invention. Examples of such osmolytes and oncotic agents include carbohydrate compounds, gelatine, serum proteins such as albumin or mixtures thereof. Examples of suitable carbohydrate compounds are sorbitol, xylitol, dextrose, polydextrose, modified and unmodified starch such as hydroxyethyl starch (HES) or mixtures of these carbohydrate compounds. These carbohydrates may usually be present in a concentration of up to about 10 % (w/v). For example, a typical concentration of hydroxyethyl starch is 6 % (w/v). If another oncotic agent such as geiatine is chosen as additive, it is typically present in an amount up to about 3.5% or 4 %

As already mentioned, the composition of the invention can be used in the large variety of therapeutic applications. It may, for example, be used in the treatment of a disease or condition selected from coronary diseases, brain disorders, organ failure, obesity, and acute hemodynamic distress due to medical and surgery. The composition can also be used for resuscitation and also for operative/postoperative treatment of patients. The composition of the invention can thus be used in emergency cases (for example, for the treatment of an increased intracranial pressure as discussed in detail below), as an agent in intensive care units (ICU) as well as parenteral food supplement for obese or hypercatabolic patients.

One therapeutic application of particular interest is the use of the pharmaceutical composition of the invention for the treatment of a brain disorder. In this case, only lactate and/or lactic acid needs to be present in a composition of the invention. Examples of such brain disorders are traumatic brain injury, cerebral ischemia or non-traumatic brain injury, metabolic disorders associated with brain dysfunction and complications associated with surgery.

In one embodiment the traumatic brain injury is closed or open craniocerebral trauma (CCT). To the surprise of the inventors, it was found that the pharmaceutical composition of the invention is not only able to significantly reduce an increase in the intracranial pressure (ICP) which is caused by the traumatic brain injury but that the efficiency exceeds that of mannitol, which is the standard osmotherapeutic approach for lowering an increased ICP.

In addition, the composition of the invention can also be administered to a patient that suffers from a non-traumatic brain injury such as stroke or cold-lesion or to a patient having a metabolic disorder associated with brain dysfunction such as hepatic or hypoglycemic coma. Due to its strong osmotic effect, the composition of the invention is also useful for the treatment of any (intracellular) brain edema caused by a traumatic or non-traumatic brain injury (disorder) so this edema is either reduced or prevented.

Examples of cardiovascular diseases or coronary diseases that can be treated with the composition of the invention are myocardial ischemia, cardiac dysfunction, cardiac and vascular complications of diabetes, acute infarction, ischemic reperfusion injury, or complications of arteriosclerosis to name a few.

As the composition generally exerts an anti-ischemic effect, it can also be used in the treatment of a patient suffering from the failure of any organ. Examples of specific organ failures that can be treated include, but are not limited to renal failure, liver failure or heart failure. In addition, it is for example also possible to treat cardiogenic shock which is caused by heart failure with the composition of the invention.

The composition of the invention has also been found to be useful for the treatment of any form of acute hemodynamic distress. This acute stress may for example, be caused by polytrauma, post-operative situations, septic shock, respiratory diseases, or acute respiratory distress syndrome.

In accordance with the above disclosure, a composition disclosed here is usually administered as a fluid. For this purpose, any suitable way of administering a fluid to a patient can be used. Preferably, the composition is administered parenterally by infusion or injection (for example by intravenous, intramuscular or intracutanous administration). For intravenous administration the composition of the invention may be given as bolus infusion or bolus injection. A typical daily maximum dosage of lactate is about 4.5 to 7.5 mmol/kg body weight/day, or calculated with a body weight of 70 kg 0.315 to 0.525 mol lactate/day. In case a composition of the invention containing 500 mM lactate is used for a treatment of a patient as described here, an amount of 4.5 mmol/kg body weight/day may be administered by bolus infusion in 15 to 20 min using a dosage of 3 to 5 ml solution/kilogram body weight. Other dosages may of course also be used, depending on the specific condition and patient to be treated. For treatment of stroke, a typical dosage of a 500 mM lactate containing composition is 10 ml solution/kg body weight/hour, i.e. 5 mmol/kg body weight/hour. Accordingly, if a 1 M lactate solution is used, the same amount of lactate can be infused at a rate of 5 ml/kg body weight/hour. If polylactate is employed in the composition of the invention, oral administration is a preferred route.

The invention further relates to a method of preparing a pharmaceutical composition containing 250 to 2000 millimoles per liter of lactic acid or the lactate, 2 to 10 millimoles per liter of potassium and, if present, also 2 to 5 millimoles per liter of calcium. This method comprises in one preferred embodiment providing respective amounts of sodium lactate or lactic acid, potassium chloride and optionally, calcium chloride and dissolving the compounds in a pharmaceutically acceptable solvent. In this respect, it is noted that the ingredients necessary for the preparation of a liquid composition of the invention, for example, sodium lactate, lactic acid, calcium chloride and potassium chloride can also be mixed as solids and this mixture is then dissolved in a pharmaceutically acceptable solvent only prior to its administration to a patient in need thereof. Accordingly, a pharmaceutical composition comprising lactate or lactic acid, and potassium (and optionally also any additional ingredients such as calcium or magnesium or an osmolytic agent) in solid form is also within the scope of the present invention. In some circumstances, for example, if storage room is limited, it might even be of advantage to prepare a solid mixture of the components of the composition of the invention, and prepare a liquid form thereof, only when needed.

In principle, every suitable combination of compounds that yield a composition having the desired content can be used for preparing the composition of the invention. For example, a composition can be prepared from lactic acid, sodium lactate, calcium chloride (x 2 H₂O), and potassium chloride. Alternatively, a mixture of calcium lactate, sodium lactate, and sodium chloride could also be used for preparing a composition of the present invention.

The solvent can be any suitable pharmaceutical acceptable solvent, for example, water, or a mixture of water with an organic solvent such as ethanol, as long as this solvent is able to dissolve the solid components, in particular of the composition in the specified amounts. Typically, the solvent is deionised, single or double distilled or micro-filtered water the purity of which is acceptable for pharmaceutical applications. The fluid composition so prepared can be treated further, for example, by heat sterilisation or sterile-filtration before administered to a patient. An example of a preferred solvent/pharmaceutical carrier used for the preparation of the composition of the invention is sterile Water for Injection (WFI) as classified by the United States Pharmacopiea (USP).

The invention is further illustrated by the attached Figures and the following non-limiting Examples.

Figure 1 shows the change in the intracranial pressure of a group of 12 patients suffering form brain trauma after receiving an infusion of the composition of the invention (closed squares, containing 500 mmol/l lactate, 9.4 mmol/l Cl, 4 mmol/l K, 2.7 mmol/l Ca and 500 mmol/l Na) compared to a group of 12 patients also suffering from brain trauma who are being treated with the conventional mannitol therapy (open squares).

Figure 2 shows the survival rate of the group of 12 patients suffering from brain trauma that are treated with a composition of the invention in comparison to the group of 12 patients having received treatment with mannitol.

Figure 3 shows the change in the osmolarity of the blood and the change in the hematocrite (Hte) and the hemoglobine (Hb) concentration in the blood for the treatment with a composition of the invention (open bars) compared to the conventional mannitol treatment (closed bars).

Figure 4 shows the change of cerebral perfusion pressure for the group of 12 patients suffering from brain trauma that are treated with a composition of the invention (open bars) compared to a group of patients having received treatment with mannitol (closed bars).

Figure 5 shows the effects of a composition of the invention (containing 500 mmol/l lactate, 9.4 mmol/l Cl, 4 mmol/l K, 2.7 mmol/l Ca and 500 mmol/l Na, open circles) on the Mean Arterial Pressure (MAP, Fig. 5A), Heart Rate (HR in beats per minute, Fig. 5B), the Cardiac Index (Cl, Fig. 5C), and the Systemic Vascular Resistance Index (SVRI, Fig. 5D) when administered to a group of 110 patients after cardiac surgery compared to the administration of Ringer's lactate (open squares) to a control group of patients that also comprised 110 individuals.

Figure 6 shows the effect of the composition administered to the 110 patients after cardiac surgery on the Pulmonary Vascular Resistance Index (PVRI) compared to the effect of the administration of Ringer's lactate.

Figure 7 shows the effect of the composition administered to the 110 patients after cardiac surgery on the capillary pulmonary wedge pressure compared to the effect of the administration of Ringer's lactate (open squares).

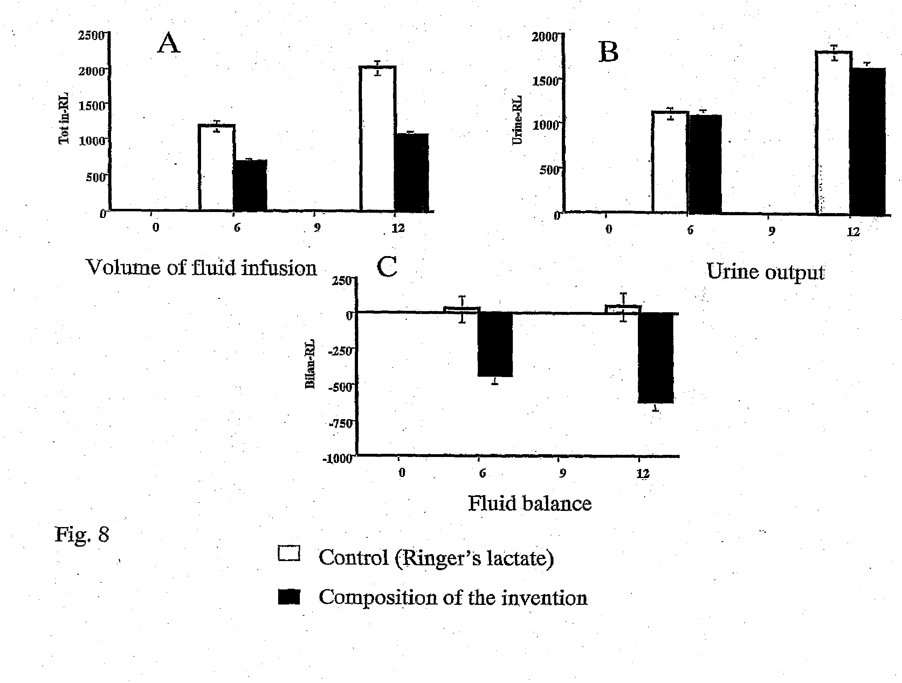

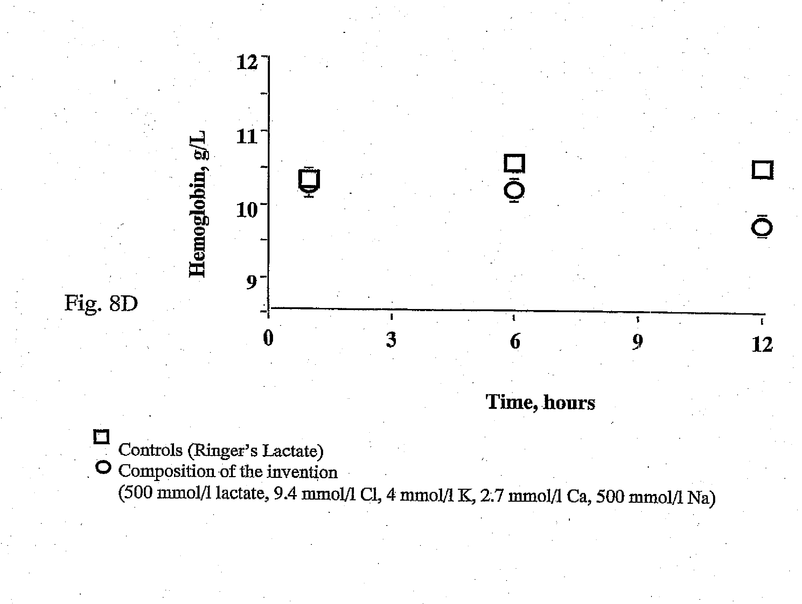

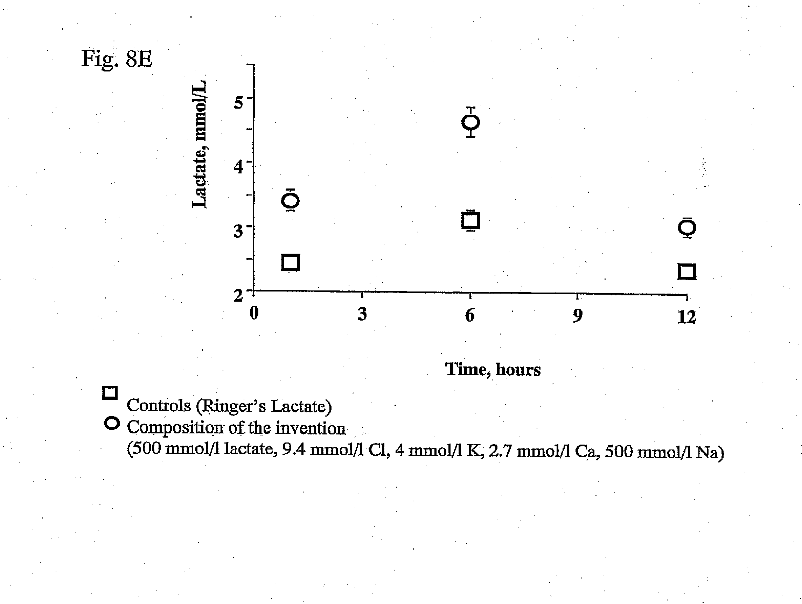

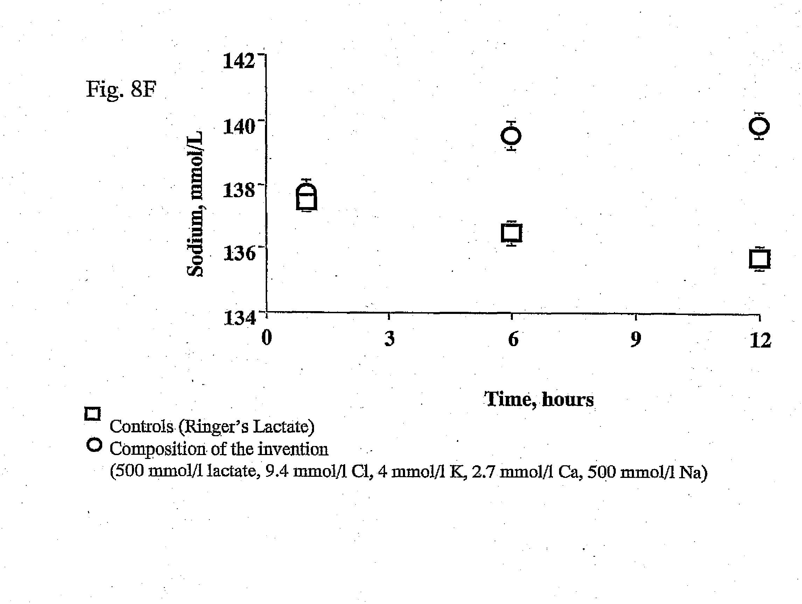

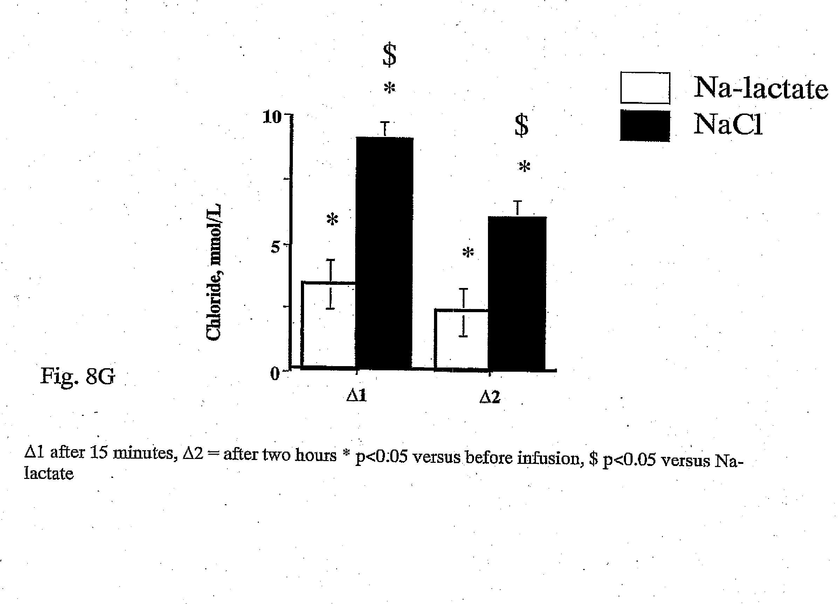

Figure 8 shows the total volume of the hypertonic lactate composition of the invention (closed bars), infused to the 110 patients compared to the total volume of infusion of Ringer's lactate (open bars) as well as the urine output (Fig. 8A), the fluid balance (Fig. 8C) of the 110 patients in each of the two groups, whereas. Figure 8D, E and F show the hemoglobine (Hb) content (Fig. 8D), the lactate blood concentration (Fig. 8E) and the sodium concentration (Fig. 8F) in the course of the infusion for the composition used in this study of post operative patients (open circles) compared to Ringer's lactate (open squares). Figure 8G shows the change in chloride blood concentration in mmol/L (before and after infusion) in two groups of each 40 patients receiving an infusion either of a sodium lactate containing composition of the invention (2.5 mmol/kg body weight over 15 minutes; open bars) or a sodium chloride containing composition (2.5 mmol/kg body weight over 15 minutes, closed bars).

Figure 9 shows the effect of lactate ,(5 mM in perfusion medium, closed squares) in an *in vitro* model for ischemia (perfused rat hearts) on the rate pressure product (RRP, Fig. 9A), the diastolic pressure (Fig. 9B) and the heart rate (beats per minute) compared to perfusion medium [control] containing 5.5 mM glucose and a 0.2 mM hexanoic acid.

### Example.1: Preparation of a pharmaceutical composition

500 litre of a composition having the concentrations: 500 mmol/L lactate, 9.4 mmol/L chloride (Cl), 4 mmol/L potassium (K), 2.7 mmol/L calcium (Ca), and 500 mmol/L sodium (Na), (which results in an osmolarity of 1008 mmol/L) were prepared from using sodium-L-lactate solution (50 weight % ), CaCl₂ x 2 H₂O (96.5 % pure), KCl (99.85 % pure) and water for injection as follows:

400 kg of water for injection are filled into a sterilized mixing tank at a temperature of 30 to 40 °C. Then, 103.6 kg of the calcium chloride, 56.2 kg of the lactate solution and 150 kg potassium chloride are continuously added under stirring. After the addition of the ingredients is completed, mixing is continued for another 45 minutes at 38 °C. Then, the remaining 56.60 kg water of injection were added in order to yield the composition having the above-mentioned concentrations.

### Example 2: Treatment of increased intracranial pressure caused by brain trauma

in this study, the pharmaceutical composition as prepared in Example 1 (containing 500 mmol/L lactate, 9.4 mmol/L chloride (Cl), 4 mmol/L potassium (K), 2.7 mmol/L calcium (Ca), and 500 mmol/L sodium (Na) was used for the treatment of patients suffering from brain injury (brain trauma). In this study, patients whose demographic data is shown in Table 1 suffering from an severe head injury (with a Glasgow Coma Score (GCS) ≤ 9 or an abnormal cerebral computer tomography (CT) scan) were included and were either treated with the above-mentioned composition or a conventional mannitol solution (20 % w/v) if they showed an intracranial pressure (ICP) of ≤ 20 mm Hg (which is considered to be life-threatening). For this purpose, the composition of the invention or the mannitol was administered (infused) to a group of each twelve patients and the ICP was measured every 15 minutes. If the ICP of a patient increased to 30 mm Hg or failed to drop by more than 5 mm Hg, the treatments were exchanged, meaning if mannitol treatment of a patient failed, the patient received an infusion of the hypertonic lactate solution in accordance with the invention, and vice versa.

**Table 1: demographic data (means ± sem.) of treated patients**

| | group treated with mannitol | group treated with hypertonic lactate |
|---|---|---|
| age (years) | 35±4 | 39.6 ± 4.9 |
| gravity score (IGS II) | 43.8 ± 3.6 | 48.7 ± 2.6 |
| GCS | 5.4±0.5 | 5.2±0.5 |
| CT scan (TDB) | 3 ± 0.2 | 5.2 ± 0.5 |
| ICH episodes | 25 | 26 |

| | | |
|---|---|---|
| GCS = Glasgow coma scale, ICH = Intracranial Hypertension | | |

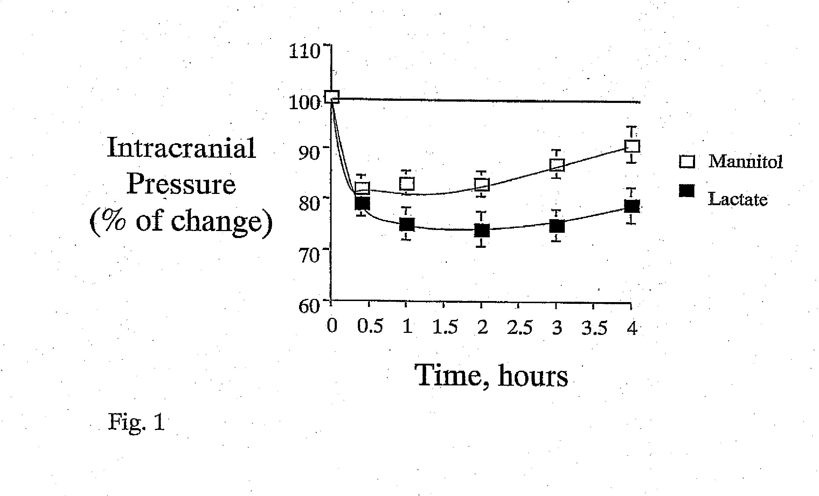

The results of this study, in which 12 patients were either treated with mannitol or the hypertonic lactate solution of the invention, are shown in Figures 1 to 4 as well as in Tables 2 to 5.

**Table 2: Results of treatment**

| | group treated with mannitol, n= 11 | group treated with hypertonic lactate, n=13 |
|---|---|---|
| number of survival | 7 out of 11 | 12 out of 13 |
| number of cross over | 4 out of 11 | 6 out of 13 |
| number of successful treatment (all treatments) | 14 out of 25 | 19 out of 26 |
| number of successful treatment (only first (treatment) | 5 out of 11 | 11 out of 13 |

**Table 3: Intracranial Pressure (ICP) and CPP before and after treatment**

| | group treated with mannitol, n= 25 | group treated with hypertonic lactate, n=26 |
|---|---|---|
| ICP (mm Hg) before treatment | 31 ±1.8 | 31.9 ±2.5 |
| ICP (mm Hg) after treatment | 25.7±1.7** | 25.2±1.9** |
| CPP (mm Hg) before treatment) | 67.6 ±2.2 | 65.8 ±2.3 |
| CPP (mm Hg) after treatment | 73.2 ±2.4 | 72.3 ±2.3 |

| | | |
|---|---|---|
| ** p ≤ 0.01 (paired T-test, comparison within the same group before and after treatment) | | |

**Table 4:**

| | group treated with mannitol, n= 25 | group treated with hypertonic lactate, n=26 |
|---|---|---|
| Osmotic Pressure (mosm/kg) before treatment | 299 ±2.6 | 298.5 ±1.6 |
| Osmotic Pressure (mosm/kg) after treatment | 302 ±2.5** | 300.0 ±1.9** |
| Arterial Hte (%) before treatment | 32.7 ±1.1 | 32.0 ±0.9 |
| Arterial Hte (%) after treatment | 31.6 ± 1.1** | 31.0 ±0.9 |

**Table 5**

| | group treated with mannitol, n= 25 | | group treated with hypertonic lactate, n=26 | |
|---|---|---|---|---|
| | before | after | before | after |
| pH arterial | 7.39± 0.02 | 7,39± 0.02 | 7.38± 0.02 | 7.42± 0.01** |
| pH venous | 7.34± 0.01 | 7.34± 0.01 | 7.33± 0.01 | 7.37±.0.01** |
| total CO₂ (mm arterial | Hg) 21.5 ±0.6 | 21.6± 0.6 | 23.2 ± 0.6 | 24.0± 0.7**^{##} |
| total CO₂ (mm Hg) arterial | 22.9±0.7 | 22.5± 0.8 | 24.5 ± 0.7 | 25.8± 0.7** |
| O₂ saturation (%) arterial | 99.3±0.8 | 99.4±0.6 | 98.9±0.3 | 99.0±0.3 |
| O₂ saturation (%) venous | 75.2± 1.6 | 77.5± 1.3** | 74.5±1.4 | 77.2± 1.6** |

| | | | | |
|---|---|---|---|---|
| ** p≤ 0.01 (paired T-test, comparison within the same group before and after treatment); ^{##} p ≤ 0.01 (paired T-test, comparison within the same group before and after treatment) | | | | |

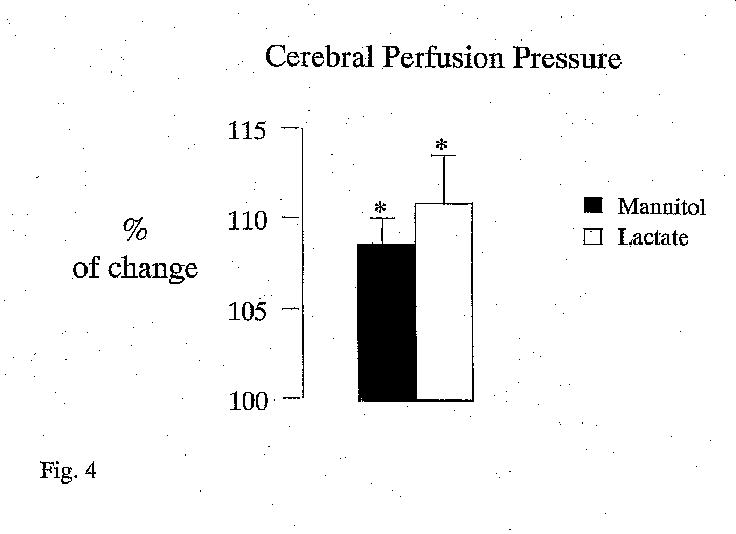

As illustrated in Fig. 1 administration of mannitol and the 0.5 M lactate solution of the present invention (which is exemplarily used to show the efficacy of a hypertonic lactate solution containing 250 mmol/l to 2400 mmol/l lactate or lactic acid) resulted in a decrease of about 20 % in the intracranial pressure within the first 30 minutes after the treatment. However, whereas mannitol showed the typical known rebound effect, meaning that the ICP steadily increased within the next 3.5 hours after the treatment to about 90 % of the initial untreated ICP, the ICP further decreased to the surprise of the inventors after the initial 30 minutes after the administration of the hypertonic lactate solution, thus showing that treatment with the hypertonic lactate solution has a prolonged effect. Likewise, as shown in Figure 4, administration of the lactate solution according to the invention lead to a greater change in the cerebral perfusion pressure (Fig. 4, see also Table 3)

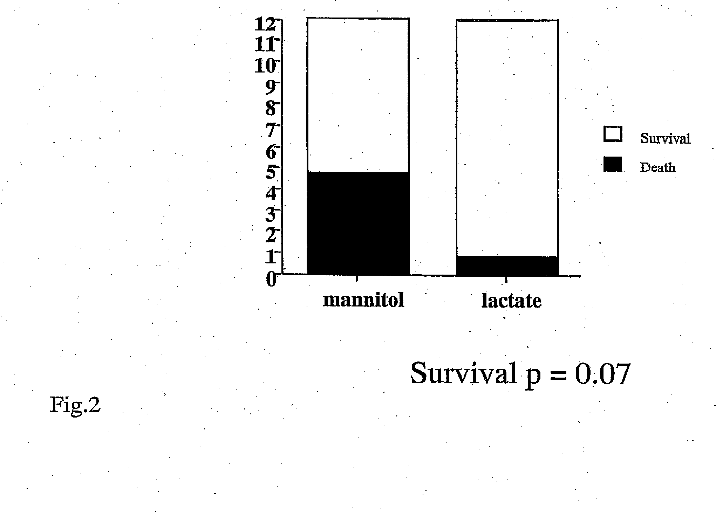

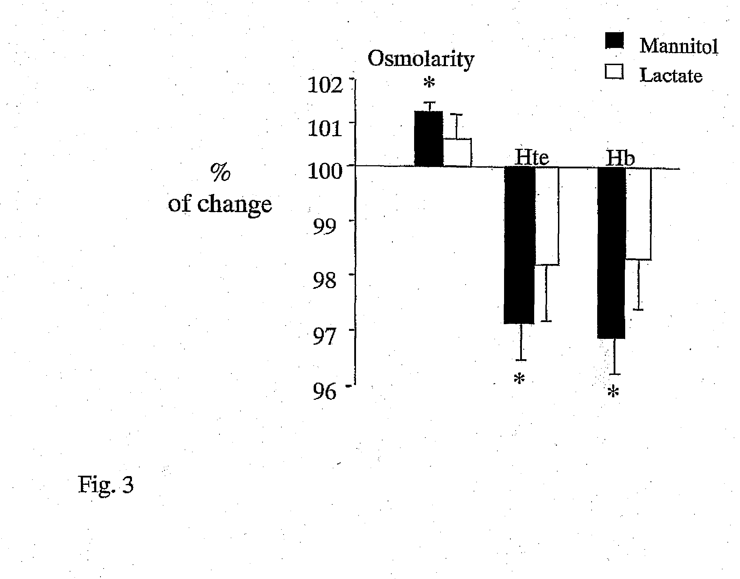

This prolonged and increased efficacy of the 0.5 M lactate solution in decreasing the ICP found here provides the further significant advantage. Namely, it is thus possible to even apply a second dose of 0.5 M lactate solution without any complications to further decrease the ICP and thus to continue the treatment - something that is hardly possible with the standard mannitol treatment. In accordance with the superior behaviour in decreasing the ICP, 11 from the 12 patients that were treated with the 0.5 M sodium lactate solution in accordance with the invention survived the brain trauma, whereas only 7 patients could be rescued by the conventional mannitol treatment (Figure 2, Table 2). Figure 3 and Table 4 show that the use of such a hypertonic lactate solution has further advantages compared to the mannitol treatment. Whereas the osmolarity of the blood changed by more than 1 % to a value of more than 101 % due to the application of the mannitol, the osmolarity of the blood changed by only 0.5 % due to the administration of the same osmolarity, of the 1 M lactate sodium solution of the invention. It is noted here that the overall change may be considered to be small. However, the relative difference of about 50 % in the change (when considered from the increase- caused by mannitol) is significant, in particular as the smaller increase in osmolarity when using the lactate solution of the invention still results in a much higher decrease in the ICP. This can also be seen from the determination of the hematocrite and hemoglobine values in Figure 3 and Table 4. A decrease in these values of more than 3 % to 97 % of the original values for the mannitol treatment Indicates a rather significant dilution of the blood due to the mannitol infusion. ln contrast to that, and despite the improved effectiveness, the application of the same volume of the 0.5 M sodium lactate only resulted in a decrease in the hematocrite and hemoglobine level to about 98.5 %. This means that hypertonic lactate such as a 0.5 M solution (or any other concentration in the range of 250 to 2000 mM as specified herein) has no such dilution effect and more importantly, that less infusion fluid needs to administered. This in turn means that the side effects due to the additional fluid uptake to the patient who is already severely affected and weakened by the traumatic brain injury, are significantly lower. This in turn is also associated with a much lower risks of (further) brain edema caused by the treatment.

Without wishing to be bound by theory, the inventors believe that the advantages of lactate are due to a different mode of action compared to mannitol. Mannitol cannot enter the affected brain cells and does not get metabolised. Thus, mannitol exerts its effect exclusively by an osmotic effect, meaning, as long as the osmotic pressure in the blood stream-extracellular compartment is higher than the ICP, water will flow from the intracellular compartment into the extracellular compartment and thus into the blood stream-extracellular compartment due to the osmotic pressure difference, When mannitol is removed from the blood stream through the urine, the osmotic pressure difference gets smaller, causing an reflux of water back into the cellular compartment and thus the known rebound effect. In this respect, it is also noted that purely relying on this pressure difference is the reason why mannitol can usually only be administered two times since the difference in the osmotic pressure of the blood and the brain gets smaller by each application. In contrast to this, the inventors believe that lactate is taken up and metabolised by any mitochondria-containing cells, including in the brain brain cells within the first about 4 hours after the application. However, only the lactate anion is metabolised (the end-product of the metabolic pathway are carbon dioxide and water), whereas the respective counter-ion such as sodium remains in the extracellular fluid. In order to achieve electroneutrality, chloride is transported together with water out of the intracellular space. This effect was shown in two groups of 40 patients (see Fig 8G) investigating the effect on plasma chloride concentration after of infusion of equimolar amount of sodium-lactate and sodium-chloride (2.5 mmol/kg of BW during 15 minutes). The rise in chloride concentration in the blood was larger in the sodium-chloride group as compared to sodium-lactate. However, there was a significant rise in chloride concentration after sodium-lactate as well indicating the chloride was driven from intracellular to extracellular fluid since in the case of sodium-lactate no chloride was infused. Thus, when applying a hypertonic lactate composition of the invention, the loss of cellular water is due to a loss of the cellular osmolarity. Hence, there will be no rebound effect and accordingly, this also provides for the possibility to give a second dose of lactate. Finally, it is noted that the consumption of the lactate anion and the increase of the sodium concentration in the body in addition is useful in preventing an acidosis.

### Example 3: Post-operative treatment of patients after cardiac surgery

The composition used in Example 1 was also employed for the resuscitation of post-operative patients that underwent elective coronary artery bypass grafting (CABD). 110 individuals were included in this study and the effectiveness of the composition was compared with the one of Ringer's lactate composition (130 mmol/l Na⁺, 5.4 mmol/l K⁺, 1.85 mmol/l Ca²⁺, 27 mmol/l lactate and 12 mmol/Cl). Infusion was performed for six hours, wherein the volume of the infused fluid was regulated by the medical personnel such that mean arterial pressure (MAP), central venous pressure (CVP), cardiac index and pulmonary capillary wedge pressure (PCWP) were maintained at a predetermined value.

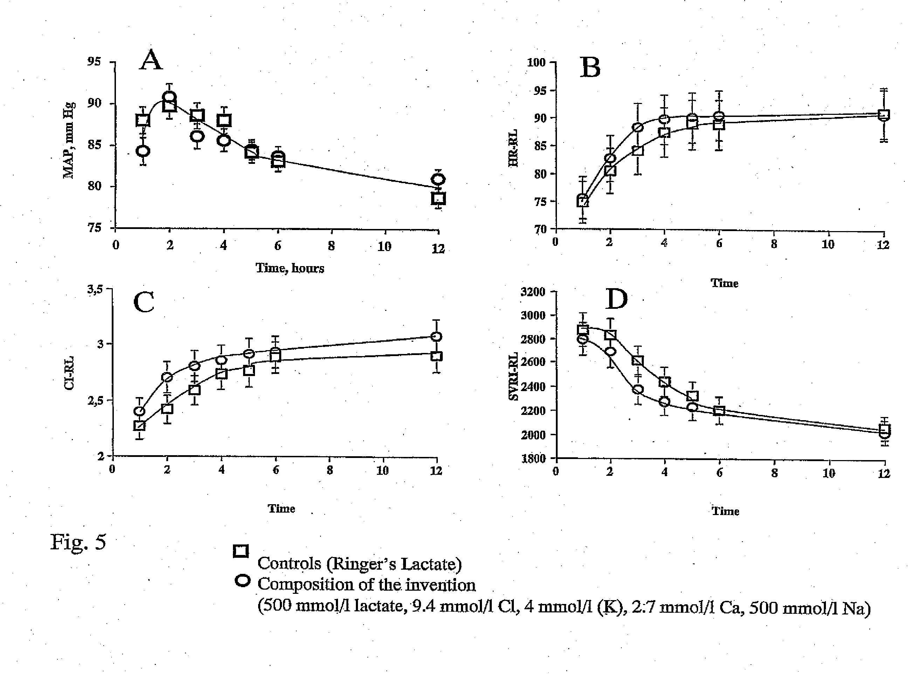

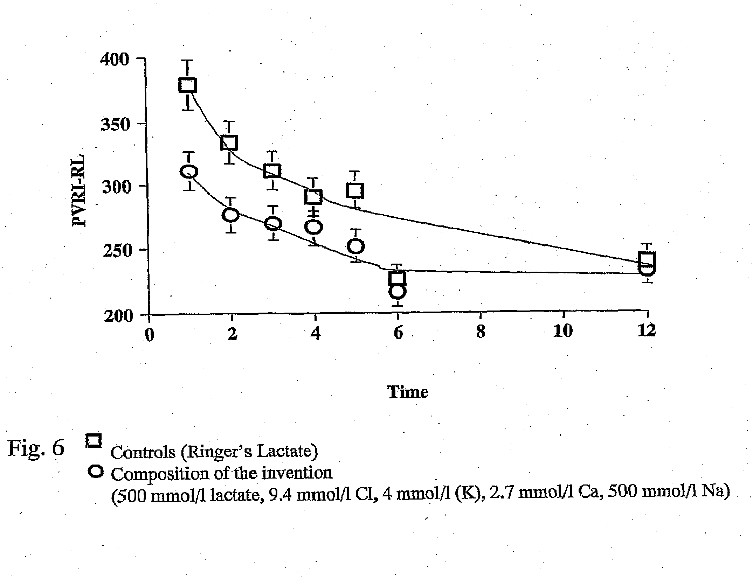

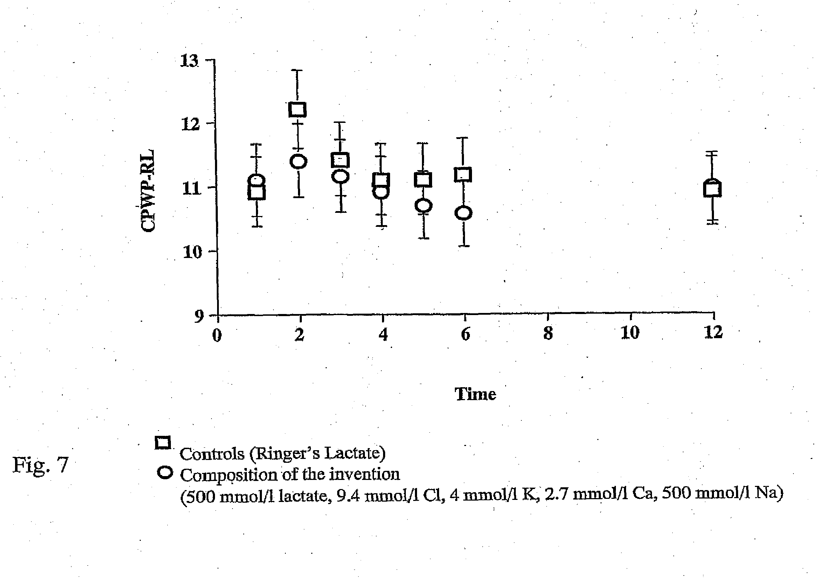

The following results were obtained from this study. Measurement of the mean arterial pressure (mm Hg) and of the heart rate (beats per minute) did not reveal any significant difference between the infusion of the hypertonic lactate, solution of the invention and conventional Ringer's lactate (see Figures 5A and 58). However, the Cardiac Index (Cl), which measures the amount of blood pumped by the heart, per minute, per meter square of body surface area was significantly higher, when the 0.5 M lactate solution was infused (Figure 5C). At the same time the systemic vascular resistance (SVRI), which is a measurement of resistance or impediment of the systemic vascular bed to blood flow, was decreased within the 6 hours of infusion (Figure 5D). This means, that infusion of the composition of the invention does not only lead to a better performance of the heart, and thus to a stabilization of the patient, but also results in a decrease in the resistance of the blood flow, and to an overall positive synergistic effect in the post-operative treatment. Furthermore, as illustrated in Figure 6, the infusion of the hypertonic solution of the invention also yields in a significantly lower pulmonary vascular resistance as evident from the pulmonary vascular resistance index (PVRI, a measurement of resistance or the impediment of the pulmonary vascular bed to blood flow), which renders the composition of the invention to be a very suitable means for the resuscitation and treatment of patients suffering from conditions such as acute (or adult) respiratory distress syndrome (ARDS), acute lung injury (ALI), interstitial lung disease (ILD) or severe acute respiratory syndrome (SARS). In addition, the filling of the patients was assessed by monitoring the Capillary Pulmonary Wedge Pressure (CPWP). As shown in Figure 7, the CPWP was identical in both patient groups during the infusion of the respective solution, indicating the same efficiency of patient's filling.

Figure 8 Illustrates further advantages of the composition of the invention compared to Ringer's lactate. The total volume infused within the first 12 hours after the heart surgery is significantly smaller (about 1200 ml) for the composition of the invention compared to 2000 ml of Ringer's lactate needed (Figure 8A), whereas the urine output of the patients is almost identical with about 1800 ml after 12 hours (Figure 8B). As a consequence, the fluid balance is negative for the patients being infused with the hypertonic lactate solution of the invention (Figure 8C), which means that the risks of developing edema that can in turn cause organ dysfunction, for example, is significantly reduced. This property of the composition of the invention is particularly advantageous since traditionally a large volume of fluid (such as Ringer's lactate) is required during surgery to maintain the patients mean arterial pressure (MAP) so that patient are always edematous after surgery. So, the composition of the invention does not only decrease the edema (or the risk of developing edema) but at the same time does also not decrease the hemodynamic state of the patient.

In order to determine the fluid flow within the body, the hemoglobine content in the blood was determined. As illustrated in Figure 8D, the hemoglobine content decreases in the course of the infusion of the hypertonic lactate composition of the invention, whereas it remains constant in the case of administration of Ringer's lactate. Since the bleeding state and the blood transfusion did not differ in the two groups of patients, this mild dilution effect during the infusion of the lactate composition results from the transfer of fluid from the surrounding tissue to the blood stream. In this respect, it is noted that as shown in Figure 8E, the lactate concentration in the blood (blood lactate level) was higher in the group receiving the hypertonic lactate composition of the invention only during the first six hours of post operative treatment, i.e. only during infusion of the lactate composition. Furthermore, as shown in Figure 8F, the sodium concentration in the blood was within the normal concentration range when the lactate composition of the invention was infused. In contrast to that, administration of Ringer's lactate composition was associated with a sodium blood concentration that is below the normal physiological concentration.

### Example 4: Suitability of lactate for the treatment of ischemia

In order to determine the suitability of lactate for the treatment of conditions such as ischemia-reperfusion, isolated rat harts were perfused either with perfusion medium containing 5 mM lactate or with a perfusion medium (control) containing 0.2 mM of hexanoic acid and 5.5 mM glucose in accordance with the protocol described by Garnier A. et al., J Mol Cell Cardiol 1996,28, 1671-1682.

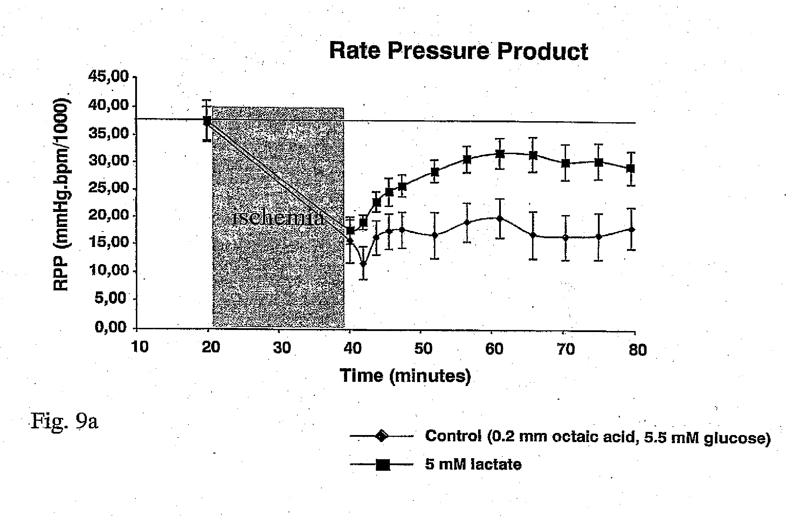

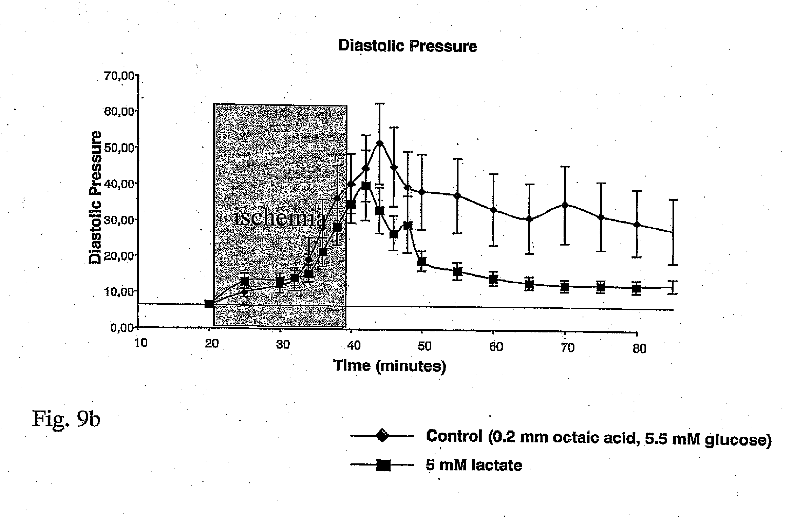

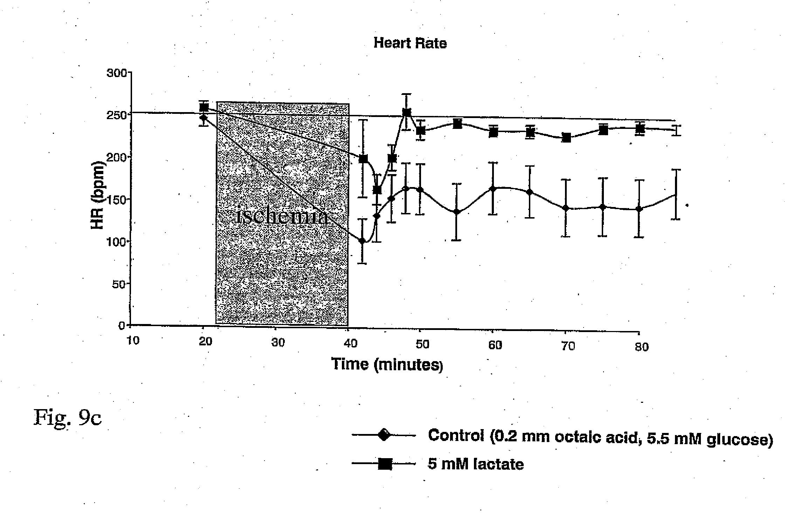

As can be seen from Figure 9, after 40 minutes of separation of the rat hearts (in order to simulate ischemia), perfusion of the lactate over a period of 40 minutes resulted in a significantly higher and prolonged increase of the rate pressure product (Figure 9A) and the heart rate (Figure9C) compared to the control. Furthermore, as shown in Fig. 9B, the diastolic pressure significantly decreased upon infusion of the lactate solution compared to the control, showing that the decompression of the heart greatly improves by the administration of the lactate solution, thereby preventing or reversing the formation of" stone heart". Accordingly, these data show that the hypertonic lactate solution described here can be advantageously used for the treatment of ischemic reperfusion injury,myocardial ischemia, or any other form of cardiac dysfunction, for example.

### Further preferred embodiments of the present invention are given in the following paragraphs:

A first further preferred embodiment of the present invention is a pharmaceutical composition containing 250 to 2400 millimoles per liter of lactic acid or lactate, and 2 to 10 millimoles per liter of potassium.

In a first aspect of the first further preferred embodiment of the present invention the concentration of lactic acid or lactate is in the range of 500 to 1500 millimoles per liter. Said concentration of lactic acid or lactate may further be in the range of 800 to 1200 millimoles per liter. Said concentration of lactic acid or lactate may further be 1000 millimoles per liter.

In a second aspect of the first further preferred embodiment of the present invention sodium (Na) is used as counter-ion for the lactate.

In a third aspect of the first further preferred embodiment of the present invention the potassium concentration is in the range of 2.5 to 6 millimoles per liter. Further, chloride (Cl) may be present as counter-ion for the potassium and the calcium

The fourth aspect of the first further preferred embodiment of the present invention forms a second further preferred embodiment of the present invention In said second further preferred embodiment of the present invention the composition further comprises 2 to 5 millimoles per liter of calcium.

In a first aspect of the second further preferred embodiment of the present invention the calcium concentration is in the range of 2.5 to 4 millimoles per liter. Further, chloride (Cl) may be present as counter ion for the potassium and the calcium. ,

In a second aspect of the second further preferred embodiment of the present invention chloride (Cl) is present as counter-ion for the potassium and the calcium.

In a fifth aspect of the first further preferred embodiment of the present invention said lactate is L-lactate.

In a sixth aspect of the first further preferred embodiment of the present invention the composition is an aqueous solution.

In a third aspect of the second further preferred embodiment of the present invention the composition has the concentrations 1000 millimoles per liter lactate, 9.4 millimoles per liter chloride (Cl), 4 millimoles per liter potassium (K), 2.7 millimoles per liter calcium (Ca), and 1000 millimoles per liter sodium (Na).

In a fourth aspect of the second further preferred embodiment of the present invention the composition has the concentrations 1000 millimoles per liter lactate, 8.9 millimoles per liter chloride (Cl), 3.5 millimoles per liter potassium (K), 2.7 millimoles per liter calcium (Ca), and 1000 millimoles per liter sodium (Na).

In a fifth aspect of the second further preferred embodiment of the present invention the composition has the concentrations 500 millimoles per liter lactate, 9.4 millimoles per liter chloride (Cl), 4 millimoles per liter potassium (K), 2.7 millimoles per liter calcium (Ca), and 500 millimoles per liter sodium (Na).

In a sixth aspect of the second further preferred embodiment of the present invention the composition has the concentration 500 millimoles per liter lactate, 8.9 millimoles per liter chloride (Cl), 3.5 millimoles per liter potassium (K), 2.7 millimoles per liter calcium (Ca), and 500 millimoles per liter sodium (Na).

A third further preferred embodiment of the present invention is a composition further comprising an osmolyte selected from the group consisting of a carbohydrate compound, gelatine, a serum protein and mixtures thereof.

In a first aspect of the third further preferred embodiment of the present invention the carbohydrate compound is dextrose, polydextrose, hydroxyethyl starch, sorbitol, xylitol or a mixture thereof.

A fourth further preferred embodiment of the present invention is the use of a pharmaceutical composition as described above in the treatment of a disease or condition selected from the group consisting of cardiovascular diseases, brain disorders, organ failure, obesity, resuscitation, and acute hemodynamic distress due to medical and surgery.

In a first aspect of the fourth further preferred embodiment of the present invention said brain disorder is selected from the group consisting of traumatic brain injury, cerebral ischemia or non-traumatic brain injury, metabolic disorders associated with brain dysfunction and complications associated with surgery. Said traumatic brain injury may be closed or open craniocerebral trauma. Further, an increased intracranial pressure caused by the traumatic brain injury may be decreased. Said non-traumatic brain injury may be stroke or cold-lesion. Said metabolic disorder associated with brain dysfunction may be hepatic or hypoglycemic coma. Further, the brain edema caused by the brain disorder may be reduced or prevented.

In a second aspect of the fourth further preferred embodiment of the present invention the cardiovascular disease is selected from the group consisting of myocardial ischemia, cardiac dysfunction, cardiac and vascular complications of diabetes, acute infarction, ischemic reperfusion injury, and complications of arteriosclerosis.

In a third aspect of the fourth further preferred embodiment of the present invention the organ failure is renal failure, liver failure or heart failure. Said the heart failure may further cause cardiogenic shock. Said acute hemodynamic distress may be caused by polytrauma, post-operative situations, septic shock, respiratory diseases, or acute respiratory distress syndrome.

In a fourth aspect of the fourth further preferred embodiment of the present invention the composition is administered by infusion or injection.

A fifth further preferred embodiment of the present invention is a method of preparing a pharmaceutical composition containing 250 to 2400 millimoles per liter of lactic acid or lactate, and 2 to 10 millimoles per liter of potassium, wherein the method comprises providing respective amounts of lactic acid or potassium lactate, and potassium chloride and dissolving the compounds in a pharmaceutical acceptable solvent.

In a first aspect of the fifth further preferred embodiment of the present invention said method further comprises providing a respective amount of calcium chloride to yield a calcium concentration of 2 to 5 millimoles. Said solvent is deionized or distilled water

In a second aspect of the fifth further preferred embodiment of the present-invention the solvent is deionized or distilled water.

A sixth further preferred embodiment of the present invention is a use of a composition containing 250 to 2400 millimoles per liter of lactic acid or lactate for the treatment of a brain disorder.

In a first aspect of the sixth further preferred embodiment of the present invention said brain disorder is selected from the group consisting of traumatic brain injury, cerebral ischemia or non-traumatic brain injury, metabolic disorders associated with brain dysfunction and complications associated with surgery. Said traumatic brain injury may be closed or open craniocerebral trauma. Further, an increased intracranial pressure caused by the traumatic brain injury may be decreased.

In a second aspect of the sixth further preferred embodiment of the present invention the non-traumatic brain injury is stroke or cold-lesion.

In a third aspect of the sixth further preferred embodiment of the present invention the concentration of lactic acid or lactate is in the range of 400 to 1500 millimoles per liter. Said concentration of lactic acid or lactate may be in the range of 500 to 1500 millimoles per liter. Said concentration of lactic acid or lactate may be in the range of 800 to 1200 millimoles per liter. Said concentration of lactic acid or lactate may be 1000 millimoles per liter.

In a fourth aspect of the sixth further preferred embodiment of the present invention said lactate is L-lactate. Said composition is an aqueous solution.

In a fifth aspect of the sixth further preferred embodiment of the present invention said composition is an aqueous solution.

A seventh further preferred embodiment of the present invention is a method of treating a patient having a brain disorder, the method comprising administering to the patient a pharmaceutical composition containing 250 to 2400 millimoles per liter of lactic acid or lactate.

In a first aspect of the seventh further preferred embodiment of the present invention said concentration of lactate or lactic acid in the composition is between 250 and 2000 millimoles per liter.

A eighth further preferred embodiment of the present invention is a method of treating a patient suffering from a disease or condition selected from the group consisting of cardiovascular diseases, brain disorders, organ failure, obesity, resuscitation, and acute hemodynamic distress due to medical and surgery, the method comprising administering to the patient a pharmaceutical composition containing 250 to 2400 millimoles per liter of lactic acid or lactate, and 2 to 10 millimoles per liter of potassium

In a first aspect of the eighth further preferred embodiment of the present invention said concentration of lactate or lactic acid in the composition is between 250 and 2000 millimoles per liter.

## Claims

1. A pharmaceutical composition containing 250 to 2400 millimoles per liter of L-lactic acid or L-lactate, and 2 to 10 millimoles per liter of potassium.

2. The composition of claim 1, wherein the concentration of L-lactic acid or L-lactate is in the range of 500 to 1500 millimoles per liter.

3. The composition of claim 1 or 2, wherein the concentration of L-lactic acid or L-lactate is in the range of 800 to 1200 millimoles per liter.

4. The composition of any of claims 1 to 3, wherein the concentration of L-lactic acid or L-lactate is 1000 millimoles per liter.

5. The composition of any of claims 1 to 4, wherein sodium (Na) is used as counter-ion for the lactate.

6. The composition of any of claims 1 to 5, wherein the potassium concentration is in the range of 2.5 to 6 millimoles per liter.

7. The composition of any of claims 1 to 6, further comprising 2 to 5 millimoles per liter of calcium.

8. The composition of claim 7, wherein the calcium concentration is in the range of 2.5 to 4 millimoles per liter.

9. The composition of any of claims 6 to 8, wherein chloride (Cl) is present as counter-ion for the potassium and the calcium.

10. The composition of any of claims 1 to 9, wherein the composition is an aqueous solution.

11. The composition of any of claims 7 to 10 having the concentrations:
1000 millimoles per liter lactate,
9.4 millimoles per liter chloride (Cl),
4 millimoles per liter potassium (K),
2.7 millimoles per liter calcium (Ca), and
1000 millimoles per liter sodium (Na).

12. The composition of any of the claims 7 to 10, having the concentrations:
1000 millimoles per liter lactate,
8.9 millimoles per liter chloride (Cl),
3.5 millimoles per liter potassium (K),
2.7 millimoles per liter calcium (Ca), and
1000 millimoles per liter sodium (Na).

13. The composition of any of claims 7 to 10 having the concentrations:
500 millimoles per liter lactate,
9.4 millimoles per liter chloride (Cl),
4 millimoles per liter potassium (K),
2.7 millimoles per liter calcium (Ca), and
500 millimoles per liter sodium (Na).

14. The composition of any of claims 7 to 10 having the concentrations:
500 millimoles per liter lactate,
8.9 millimoles per liter chloride (Cl),
3.5 millimoles per liter potassium (K),
2.7 millimoles per liter calcium (Ca), and
500 millimoles per liter sodium (Na).

15. The composition of any of the foregoing claims further comprising an osmolyte selected from the group consisting of a carbohydrate compound, gelatine, a serum protein and mixtures thereof.

16. The composition of claim 15, wherein the carbohydrate compound is dextrose, polydextrose, hydroxyethyl starch, sorbitol, xylitol or a mixture thereof.

17. The use of a composition containing 250 to 2400 millimoles per liter of L-lactic acid or L-lactate as active ingredient for the manufacture of a pharmaceutical for treatment of traumatic brain injury.

18. The use of claim 17, wherein the traumatic brain injury is closed or open craniocerebral trauma.

19. The use of a composition containing 250 to 2400 millimoles per liter of L-lactic acid or L-lactate as active ingredient for the manufacture of a pharmaceutical for treatment of non-traumatic brain injury, wherein the non-traumatic brain injury is stroke or cold-lesion.

20. The use of any of claims 17 to 19, wherein the concentration of L-lactic acid or L-factate is in the range of 400 to 1500 millimoles per liter.
